# EUROPÄISCHE PATENTANMELDUNG

(11) **EP 2 545 889 A1**
(43) Veröffentlichungstag der Anmeldung: **16.01.2013**
(21) Anmeldenummer: 12005078.6
(22) Anmeldetag: 09.07.2012
(51) Int. Cl.: A61F 5/37

(54) **Fixierbandage zur Fixierung eines Patienten**

(30) Priorität: 12.07.2011 DE 202011103318 U
(71) Anmelder: Wysozki, Roman, 22763 Hamburg (DE)
(72) Erfinder: Wysozki, Roman, 22763 Hamburg (DE)
(74) Vertreter: Hauck Patent- und Rechtsanwälte

(57) **Zusammenfassung**

Fixierbandage zur Fixierung eines Patienten auf einem Bett oder einem sonstigen Lager, die im angelegten Zustand folgendes aufweist zwei Betthalterungen zur Befestigung an einem Bettgestell, einen linken ersten Gurtabschnitt und einen rechten ersten Gurtabschnitt, die jeweils ein erstes Ende, das mit einer der Betthalterungen verbunden ist, und ein zweites Ende aufweisen, wobei die beiden zweiten Enden in einem ersten Abstand voneinander auf einer Liegefläche des Betts angeordnet sind, einen linken zweiten Gurtabschnitt und einen rechten zweiten Gurtabschnitt, die jeweils ein erstes Ende und ein zweites Ende aufweisen, wobei das erste Ende des linken zweiten Gurtabschnitts mit dem zweiten Ende des linken ersten Gurtabschnitts verbunden ist, das erste Ende des rechten zweiten Gurtabschnitts mit dem zweiten Ende des rechten ersten Gurtabschnitts verbunden ist und die zweiten Enden der beiden zweiten Gurtabschnitte in einem zweiten Abstand voneinander im Rücken des Patienten angeordnet sind, einen linken dritten Gurtabschnitt und einen rechten dritten Gurtabschnitt, die jeweils ein erstes Ende und ein freies Ende aufweisen, wobei das erste Ende des linken dritten Gurtabschnitts mit dem zweiten Ende des linken zweiten Gurtabschnitts verbunden ist, das erste Ende des rechten dritten Gurtabschnitts mit dem zweiten Ende des rechten zweiten Gurtabschnitts verbunden ist und die freien Enden lösbar miteinander verbunden sind, einem vierten Gurtabschnitt, der die beiden zweiten Enden der ersten Gurtabschnitte miteinander verbindet, und einem fünften Gurtabschnitt, der die beiden ersten Enden der dritten Gurtabschnitte miteinander verbindet.

## Beschreibung

Die Erfindung betrifft eine Fixierbandage zur Fixierung eines Patienten auf einem Bett oder einem sonstigen Lager.

Aus dem Gebrauchsmuster DE 203 17 701 U1 ist eine Fixierbandage bekannt geworden, die einen flach auf das Bett aufzulegenden Bettgurt aufweist, der zur Befestigung an einem Bettgestell des Betts an jedem seiner beiden Enden eine Betthalterung aufweist. Die Betthalterungen werden jeweils schlaufenförmig um einen Holm des Bettgestells herumgeschlungen und mit einer geeigneten Verriegelung, beispielsweise einem Magnetschloss, geschlossen. Dadurch ist der Bettgurt an dem Bett befestigt. An dem Bettgurt ist ein Leibgurt befestigt, der ringförmig um die Taille des Patienten herumgelegt und mit einer weiteren Verriegelung geschlossen werden kann. Dadurch ist der Patient an der Taille auf dem Bett fixiert. Dies schützt ihn vor einem Herausfallen aus dem Bett, ohne seine Bewegungsfreiheit insbesondere an den Händen und Füßen unnötig einzuschränken. Bei derartigen Fixierbandagen erhält der Patient zusätzliche Bewegungsfreiheit dadurch, dass der Leibgurt nicht unmittelbar mit dem Bettgurt vernäht ist, sondern mit Hilfe sich überkreuzender Bänder. Dies ist in Grundzügen bereits in der Offenlegungsschrift DE 1 953 842 beschrieben. Durch die sich überkreuzenden Bänder kann der ringförmig geschlossene Leibgurt in einem gewissen Bereich auf dem Bettgurt abrollen und somit dem Patienten ermöglichen, sich auf die Seite zu drehen.

Dieser zusätzliche Bewegungsspielraum kann sich unter Umständen jedoch auch als nachteilig erweisen, insbesondere wenn der Patient infolge einer seitlichen Drehung seine Beine seitlich über eine Bettkante hinauslehnt, zu tief in den Leibgurt hineinrutscht und dann von dem Leibgurt in seiner Atmung beeinträchtigt wird.

Es ist daher bekannt geworden, die seitliche Bewegungsfreiheit durch einen zusätzlichen Quergurt, der sich von der Oberseite des Leibgurts seitlich zu den Betthalterungen erstreckt, gezielt einzuschränken, wie im Einzelnen in der Druckschrift DE 20 2006 009 390 U1 beschrieben.

Eine andere Form einer zusätzlichen seitlichen Fixierung ist in der bereits genannten Druckschrift DE 20 317 701 U1 beschrieben. Hier werden an beiden Seiten des Leibgurts zusätzliche Seitenbefestigungen angebracht, die mit den Betthalterungen verbunden werden. Für eine sichere Verwendung von Fixierbandagen mit derartigen zusätzlichen Seitenbefestigungen müssen diese immer gewissenhaft und korrekt angelegt werden. Außerdem besteht ein Risiko, dass der Patient mit einem Körperteil, insbesondere mit dem Kopf, unter eine der Seitenbefestigungen gerät.

Um die zusätzlichen Seitenbefestigungen einzusparen und die Handhabung der Fixierbandage zu vereinfachen, schlägt die Druckschrift DE 20 2006 009 392 U1 vor, den Leibgurt und den Bettgurt unmittelbar miteinander zu vernähen. Hierdurch kann zwar auf die Seitenbefestigungen verzichtet werden, der Patient wird jedoch in seiner Bewegungsfreiheit stärker eingeschränkt und es ist nicht länger möglich, ihm durch gezieltes Lockern der Seitenbefestigungen ein gewünschtes Maß an Bewegungsfreiheit einzuräumen.

Davon ausgehend ist es die Aufgabe der Erfindung, eine Fixierbandage zur Fixierung eines Patienten auf einem Bett oder einem sonstigen Lager zur Verfügung zu stellen, die dem Patienten ein definiertes Maß an Bewegungsfreiheit lässt, einfach und robust aufgebaut und einfach anzulegen ist.

Diese Aufgabe wird gelöst durch die Fixierbandage mit dem Merkmalen des Anspruchs 1. Vorteilhafte Ausgestaltungen sind in den sich anschließenden Unteransprüchen angegeben.

Die Fixierbandage dient zur Fixierung eines Patienten auf einem Bett oder einem sonstigen Lager und weist im angelegten Zustand Folgendes auf:
● zwei Betthalterungen zur Befestigung an einem Bettgestell,
● einen linken ersten Gurtabschnitt und einen rechten ersten Gurtabschnitt, die jeweils ein erstes Ende, das mit einer der Betthalterungen verbunden ist, und ein zweites Ende aufweisen, wobei die beiden zweiten Enden in einem ersten Abstand voneinander auf einer Liegefläche des Betts angeordnet sind,
● einen linken zweiten Gurtabschnitt und einen rechten zweiten Gurtabschnitt, die jeweils ein erstes Ende und ein zweites Ende aufweisen, wobei das erste Ende des linken zweiten Gurtabschnitts mit dem zweiten Ende des linken ersten Gurtabschnitts verbunden ist, das erste Ende des rechten zweiten Gurtabschnitts mit dem zweiten Ende des rechten ersten Gurtabschnitts verbunden ist und die zweiten Enden der beiden zweiten Gurtabschnitte in einem zweiten Abstand voneinander im Rücken des Patienten angeordnet sind,
● einen linken dritten Gurtabschnitt und einen rechten dritten Gurtabschnitt, die jeweils ein erstes Ende und ein freies Ende aufweisen, wobei das erste Ende des linken dritten Gurtabschnitts mit dem zweiten Ende des linken zweiten Gurtabschnitts verbunden ist, das erste Ende des rechten dritten Gurtabschnitts mit dem zweiten Ende des rechten zweiten Gurtabschnitts verbunden ist und die freien Enden lösbar miteinander verbunden sind,
● einem vierten Gurtabschnitt, der die beiden zweiten Enden der ersten Gurtabschnitte miteinander verbindet, und
● einem fünften Gurtabschnitt, der die beiden ersten Enden der dritten Gurtabschnitte miteinander verbindet.

Die Fixierbandage dient zur Fixierung eines Patienten auf einem Bett oder einem sonstigen Lager. Mit Fixierung ist einerseits eine Ruhigstellung verwirrter, unkooperativer oder nicht zurechnungsfähiger Patienten gemeint. Eingezogen ist darüber hinaus eine Lagerung von Patienten in einer bevorzugten Haltung aus anderen Gründen, beispielsweise zur Dekubitus-Prophylaxe. Das sonstige Lager kann beispielsweise eine Trage oder eine Liege sein, etwa für einen Krankentransport. Soweit in den Anspruchsmerkmalen auf Elemente des Betts wie das Bettgestell oder eine Liegefläche Bezug genommen wird, geschieht dies der Einfachheit halber stellvertretend für entsprechende Komponenten sonstiger Lager. Beispielsweise kann das Bettgestell im Falle einer Trage ein Rahmen der Trage sein und die Liegefläche des Betts kann eine Liegefläche der Trage sein, usw.

Die Betthalterungen können Gurte sein, die zur Befestigung an einem Bettgestell um einen Bettholm des Bettgestells (oder, beispielsweise, zur Befestigung an einer Trage um einen Träger eines Rahmens der Trage) herumgeführt werden. Die von jeder Betthalterung auf diese Weise gebildete Schlaufe wird mit einer geeigneten Verriegelung geschlossen. Diese ist beispielsweise ein Magnetschloss, das einen Sockel mit einem Teller und einem Schaft und einen Schließknopf aufweist. Der Schaft wird durch zwei Ösen in der Betthalterung hindurch gesteckt und mit dem Schließknopf versehen. Der Teller des Sockels kann sich unterhalb der Ösen in einer Tasche der Betthalterung befinden.

Die ersten, zweiten und dritten Gurtabschnitte sind jeweils paarweise vorhanden und werden als linke und rechte Abschnitte bezeichnet. Damit ist gemeint, dass sich die betreffenden Abschnitte im Wesentlichen an einer rechten oder linken Seite des Patienten befinden. Insbesondere können sie im Wesentlichen spiegelsymmetrisch zu beiden Seiten einer Sagittalebene des Patienten angeordnet sein. Natürlich können sich die jeweiligen Gurtabschnitte auch teilweise auf die jeweils gegenüberliegende Seite des Patienten erstrecken, was insbesondere für die sich überlappenden freien Enden der dritten Gurtabschnitte gilt.

Dass die einzelnen Gurte bzw. Enden der einzelnen Gurte miteinander verbunden sind, wie vorstehend im einzelnen angegeben, bedeutet, dass Zugkräfte von dem einen Gurtabschnitt auf den damit verbundenen Gurtabschnitt übertragen werden können. Die Verbindung kann fest ausgeführt sein, beispielsweise durch Vernähen der beiden Gurtabschnitte, insbesondere in einem Längsabschnitt, indem sich die jeweiligen Gurtabschnitte überlappen. Hier können die Abschnitte insbesondere großflächig, das heißt durch mehrere voneinander beabstandete Nähte, vernäht sein. Die Verbindung kann jedoch auf lösbar ausgeführt sein, etwa durch Schlaufen oder Ösen, durch die Enden der Gurtabschnitte hindurchgeführt oder an denen sie in sonstiger Weise befestigt sind. Darüber hinaus können miteinander in der beanspruchten Weise verbundene Gurtabschnitte auch von einem einteilig ausgebildeten Gurt gebildet sein. In diesem Fall ist die Unterteilung in unterschiedliche Abschnitte gedanklicher Art bzw. wird erst durch einen Verbindungsbereich zu einem weiteren Gurtabschnitt definiert.

Die beiden ersten Gurtabschnitte und der vierte Gurtabschnitt, der diese miteinander verbindet, liegen im Wesentlichen auf einer Liegefläche des Betts auf, beispielsweise auf einer Matratze. Sie entsprechen hinsichtlich ihrer Funktion demnach einem Bettgurt.

Die beiden dritten Gurtabschnitte sind im Bereich ihrer freien Enden lösbar miteinander verbunden, beispielsweise über ein Magnetschloss, das durch Ösen in den freien Enden der dritten Gurtabschnitte hindurchgeführt ist. Die beiden dritten Gurtabschnitte und der fünfte Gurtabschnitt, der die beiden dritten Gurtabschnitte miteinander verbindet, umschließen den Patienten ringförmig und entsprechen daher ihrer Funktion nach einem Leibgurt.

Die beiden zweiten Gurtabschnitte stellen eine Verbindung her zwischen den in diesem Sinne einen Bettgurt bildenden ersten und vierten Gurtabschnitten und den in diesem Sinne einen Leibgurt bildenden dritten und fünften Gurtabschnitten. Die Länge der zweiten Gurtabschnitte und die genaue Anordnung der Verbindungsbereiche der übrigen Gurtabschnitte legen fest, wie viel Bewegungsfreiheit dem Patienten verbleibt. Die zweiten Gurtabschnitte können eine Länge im Bereich von beispielsweise 10 cm bis 25 cm aufweisen, insbesondere im Bereich von 15 cm bis 20 cm.

Anders als bei der aus dem Stand der Technik bekannten Verbindung zwischen Bettgurt und Leibgurt über sich überkreuzende Bänder kreuzen sich die zweiten Gurtabschnitte nicht, sondern bilden, wenn die den Leibgurt bildenden Abschnitte in einem maximal möglichen Abstand von den den Bettgurt bildenden Abschnitten angeordnet werden, zwei gegenüberliegende Seiten eines Parallelogramms. Die beiden übrigen Seiten dieses Parallelogramms, insbesondere dessen parallel angeordnete Seiten, werden von dem vierten Gurtabschnitt und dem fünften Gurtabschnitt gebildet.

Diese Anordnung der einzelnen Gurtabschnitte ermöglicht einerseits eine Abrollbewegung der den Leibgurt bildenden Abschnitte auf den den Bettgurt bildenden Abschnitten, in etwa vergleichbar zu der dem Patienten möglichen Bewegung bei Verwendung sich kreuzender Bänder. Andererseits erhält der Patient zusätzlich die Möglichkeit, unabhängig von einer Drehbewegung in einem gewissen Bereich seitlich hin- und herzurutschen. Dies kann es dem Patienten selbst bei nur geringfügiger seitlicher Bewegungsfreiheit erheblich erleichtern, eine bequeme Liegeposition zu finden und den Tragekomfort der Fixierbandage dadurch beträchtlich erhöhen.

In einer Ausgestaltung weisen der fünfte Gurtabschnitt und die beiden dritten Gurtabschnitte die gleiche Breite auf. Die Breite kann beispielsweise im Bereich von 14 cm bis 22 cm liegen, bevorzugt im Bereich von 16 cm bis 20 cm. Somit weisen die den Leib des Patienten ringförmig umschließenden Gurtabschnitte eine relativ große, gleichmäßige Breite auf, was zu einem hohen Tragekomfort beiträgt.

In einer Ausgestaltung weisen die ersten Gurtabschnitte und/oder die zweiten Gurtabschnitte und/oder die dritten Gurtabschnitte eine erste Breite auf und der vierte Gurtabschnitt eine zweite Breite, die geringer ist als die erste Breite. Die erste Breite kann beispielsweise in dem vorstehend genannten Bereich der Breite der dritten Gurtabschnitte und des fünften Gurtabschnitts liegen. Der vierte Gurtabschnitt weist eine zweite, geringere Breite auf, die beispielsweise im Bereich von 4 cm bis 8 cm, insbesondere im Bereich von 3 cm bis 6 cm liegt. Diese Ausgestaltung ist materialsparend und beeinträchtigt den Tragekomfort nicht, weil der schmaler ausgeführte vierte Gurtabschnitt nicht unmittelbar mit dem Patienten in Berührung gelangt. Außerdem wird er auch bei kraftvollen Bewegungen des Patienten weniger stark belastet als die vorzugsweise breiter ausgeführten Gurtabschnitte, so dass eine ausreichende Festigkeit gewährleistet ist. Insbesondere können die ersten, zweiten und dritten Gurtabschnitte die erste Breite aufweisen.

In einer Ausgestaltung sind zwei Hauptgurte vorhanden, die jeweils einen der ersten Gurtabschnitte, einen der zweiten der Gurtabschnitte und einen der dritten Gurtabschnitte bilden und jeweils zumindest einen ersten Materialstreifen aufweisen, der durchgängig vom ersten Ende des ersten Gurtabschnitts bis zum freien Ende des dritten Gurtabschnitts verläuft. Insbesondere sind ein linker und ein rechter Hauptgurt vorgesehen, die die genannten linken bzw. rechten Gurtabschnitte bilden. Die Hauptgurte können insgesamt durchgängig vom ersten Ende des ersten Gurtabschnitts bis zum freien des dritten Gurtabschnitts ausgeführt sein, gegebenenfalls bestehend aus mehreren unterschiedlichen Lagen und/oder Materialien. Sie weisen mindestens einen ersten Materialstreifen auf, der im genannten Bereich durchgängig verläuft. Die durchgängige Ausbildung der Hauptgurte führt im Vergleich zu auf sonstige Weise miteinander verbundenen, insbesondere miteinander vernähten Gurtabschnitten zu einer besonders hohen Festigkeit. Dies ist insbesondere für die von den Hauptgurten gebildeten Gurtabschnitte wichtig, weil diese besonders großen Zugkräften ausgesetzt werden, und trägt wesentlich zu einer langen Lebensdauer der Fixierbandage bei. Die Verwendung der Hauptgurte ermöglicht auch eine besonders einfache und materialsparende Herstellung, denn die Anzahl der Verbindungen der unterschiedlichen Gurtabschnitte reduziert sich und es gibt entsprechend weniger sich überlappende Bereiche.

In einer Ausgestaltung ist mindestens ein zweiter Materialstreifen vorhanden, der durchgängig von einem freien Ende eines der dritten Gurtabschnitte bis zu einem freien Ende einer der Betthalterungen verläuft. Ein durchgängiger Verlauf bis zu einem freien Ende einer der Betthalterungen, insbesondere ein durchgängiger Verlauf von dem freien Ende einer linken Betthalterung zu dem freien Ende einer rechten Betthalterung, verbessert die Festigkeit und Belastbarkeit der Fixierbandage zusätzlich. Insbesondere kann eine gesonderte Verbindung zwischen Betthalterungen und ersten Gurtabschnitten bzw. Hauptgurten entfallen oder auf eine Verbindung zusätzlicher Materiallagen beschränkt werden.

In einer Ausgestaltung sind der erste Materialstreifen und der zweite Materialstreifen miteinander vernäht und der zweite Materialstreifen weist eine geringere Breite auf als der erste Materialstreifen. Der erste und zweite Materialstreifen können unmittelbar aneinander anliegen. Es können auch weitere Materiallagen zwischen den beiden Materialstreifen angeordnet sein. Die beiden Materialstreifen sind mindestens entlang eines Längsabschnitts miteinander vernäht, beispielsweise im Wesentlichen entlang der gesamten Länge der linken und rechten ersten Gurtabschnitte. Alternativ können der erste Materialstreifen und der zweite Materialstreifen die gleiche Breite aufweisen, beispielsweise die vorstehend genannte zweite Breite. Durch die erläuterten Maßnahmen wird eine besonders feste Verbindung der betreffenden Gurtabschnitte erzielt.

In einer Ausgestaltung ist an dem fünften Gurtabschnitt ein Schrittgurt befestigt. Hierzu kann der Schrittgurt insbesondere unmittelbar mit dem fünften Gurtabschnitt vernäht sein, beispielsweise großflächig und/oder anhand mehrerer benachbarter Nähte. Der Schrittgurt kann auch mittelbar, d.h. beispielsweise über ein Verstärkungs- oder Zwischenstück, an dem fünften Gurtabschnitt angebracht sein. Es kann auch eine lösbare Befestigung, beispielsweise mit Hilfe einer Schlaufe oder Öse, vorgesehen sein. Der Schrittgurt kann ein freies Ende mit einer Befestigungseinrichtung aufweisen, beispielsweise mit mehreren voneinander beabstandeten Ösen, die eine Befestigung des freien Endes des Schrittgurts am Leibgurt, insbesondere im Bereich der beiden lösbar miteinander verbundenen freien Enden der dritten Gurtabschnitte, ermöglicht. Der Schrittgurt kann ein Polster aufweisen, das zwischen den Beinen des Patienten angeordnet ist und den Tragekomfort verbessert und vor Verletzungen schützt. Der Schrittgurt hat die Wirkung, dass ein zu tiefes Hineinrutschen des Patienten in die Fixierbandage zuverlässig verhindert wird. Anstelle eines Schrittgurts können auch Oberschenkelgurte oder -manschetten verwendet werden, die ringförmig um jeweils einen Oberschenkel des Patienten herum angelegt werden und ebenfalls ein zu tiefes Hineinrutschen des Patienten in die Fixierbandage verhindern. Die Oberschenkelgurte oder -manschetten können auf die gleiche Art und Weise an dem fünften Gurtabschnitt befestigt werden wie für den Schrittgurt beschrieben.

In einer Ausgestaltung ist an den dritten Gurtabschnitten oder an dem fünften Gurtabschnitt eine Befestigungseinrichtung zur Anbringung eines Brust- oder Schultergurts ausgebildet. Die Befestigungseinrichtung kann beispielsweise von zwei in einem Abstand voneinander angeordneten Schlaufen gebildet sein, durch die weitere Gurte hindurchgeführt werden können. Ein auf diese Weise angebrachter Brust- oder Schultergurt kann ein Herausflüchten des Patienten aus der Fixierbandage nach oben verhindern. Alternativ kann der Brust- oder Schultergurt auch unlösbar an den dritten Gurtabschnitten oder an dem fünften Gurtabschnitt befestigt sein, insbesondere durch Vernähen. Hierzu kann der Brust- oder Schultergurt unmittelbar oder gegebenenfalls über ein zusätzliches Verstärkungs- oder Zwischenstück an den genannten Gurtabschnitten angebracht sein.

In einer Ausgestaltung ist ein Verbindungsbereich zwischen zweien der Gurtabschnitte mit einem Materialstück verstärkt, das mit den beiden Gurtabschnitten vernäht ist. Das Materialstück kann beispielsweise rechteckig oder oval sein und beispielsweise aus einem Gurtmaterial bestehen. Eine derartige Verstärkung von Verbindungsbereichen zwischen zweien der Gurtabschnitte erhöht die Belastbarkeit und Lebensdauer der Fixierbandage.

Die Erfindung wird nachfolgend anhand eines in vier Figuren dargestellten Ausführungsbeispiels näher erläutert. Es zeigen:
- Fig. 1: eine erfindungsgemäße Fixierbandage in an einen Patienten angelegtem Zustand in einer vereinfachten Schnittsdarstellung,
- Fig. 2: die Fixierbandage aus Fig. 1 in einer perspektivischen Darstellung ohne Patienten,
- Fig. 3: einen Ausschnitt der Fixierbandage aus Fig. 1 in einer perspektivischen Ansicht von unten,
- Fig. 4: einen Ausschnitt der Fixierbandage aus Fig. 1 in einer weiteren perspektivischen Darstellung.

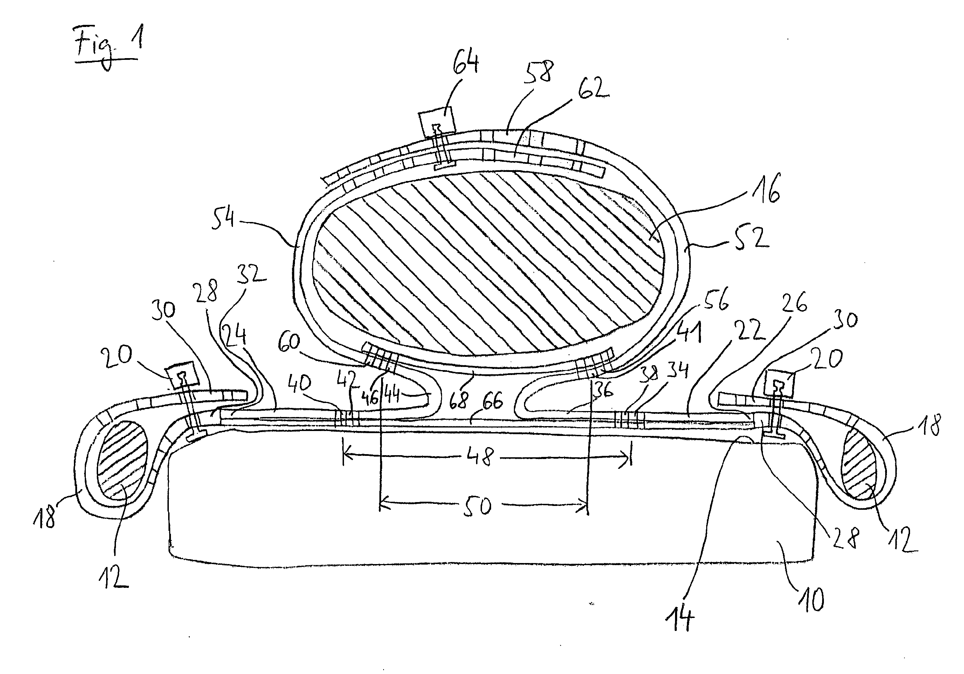

Fig. 1 zeigt im Querschnitt ein Bett mit Matratze 10 und zwei Holmen eines Bettgestells 12. Die Oberseite der Matratze 10 bildet eine Liegefläche 14, auf der ein auf dem Rücken liegender Patient 16 ruht. Der Abstand zwischen Patient 16 und Liegefläche 14 ist vergrößert dargestellt, um die dazwischen angeordneten Elemente der Fixierbandage besser erkennbar darstellen zu können. Der dargestellte Schnitt verläuft durch den Leib des Patienten im Bauchbereich und der Blick ist von unten, das heißt von den Füßen des Patienten aus, auf diese Schnittfläche gerichtet. Deshalb sind die rechts des Patienten befindlichen Elemente in der Figur links dargestellt und umgekehrt.

Die Fixierbandage weist zwei Betthalterungen 18 auf, die jeweils um einen Holm des Bettgestells 12 herumgeschlungen und mit einem Magnetschloss 20, das durch zwei Ösen der Betthalterungen 18 hindurchgeführt ist, befestigt sind.

Die Fixierbandage weist weiterhin einen linken ersten Gurtabschnitt 22 und einen rechten ersten Gurtabschnitt 24 auf. Der linke erste Gurtabschnitt 22 hat ein erstes Ende 26, das mit der linken Betthalterung 18 verbunden ist. Genauer ist das erste Ende 26 des linken ersten Gurtabschnitts 22 mit einem ersten Ende 28 der linken Betthalterung 18 verbunden. Die linke Betthalterung 18 weist außerdem ein freies Ende 30 auf. Gleiches gilt für das erste Ende 32 des rechten ersten Gurtabschnitts 24, das mit einem ersten Ende 28 der rechts des Patienten angeordneten Betthalterung 18 verbunden ist. Auch diese Betthalterung 18 weist ein freies Ende 30 auf. Die Verbindungen zwischen den Betthalterungen 18 und linken und rechten ersten Gurtabschnitten 22, 24 sind in der Figur nicht im Einzelnen dargestellt. Sie können auf unterschiedliche Weise hergestellt werden, etwa durch Vernähen überlappender Gurtabschnitte oder durch Verwendung durchgängiger Materialstreifen.

Der linke erste Gurtabschnitt 22 hat ein zweites Ende 34, das mit einem ersten Ende 38 eines linken zweiten Gurtabschnitts 36 verbunden ist. Der linke zweite Gurtabschnitt 36 hat außerdem ein zweites Ende 41. Entsprechend hat der rechte erste Gurtabschnitt 24 ein zweites Ende 40, das mit einem ersten Ende 42 eines rechten zweiten Gurtabschnitts 44 verbunden ist. Der rechte zweite Gurtabschnitt 44 hat außerdem ein zweites Ende 46.

Das zweite Ende 34 des linken ersten Gurtabschnitts 22 und das zweite Ende 40 des rechten ersten Gurtabschnitts 24 sind in einem ersten Abstand 48 voneinander auf der Liegefläche 14 angeordnet. Dieser erste Abstand 48 liegt beispielsweise im Bereich von 20 cm bis 30 cm.

Das zweite Ende 41 des linken zweiten Gurtabschnitts 36 und das zweite Ende 46 des rechten zweiten Gurtabschnitts 44 sind in einem zweiten Abstand 50 voneinander im Rücken des Patienten angeordnet. Der zweite Abstand 50 liegt beispielsweise im Bereich von 15 cm bis 25 cm.

Weiterhin weist die Fixierbandage einen linken dritten Gurtabschnitt 52 und einen rechten dritten Gurtabschnitt 54 auf. Der linke dritte Gurtabschnitt 52 weist ein erstes Ende 56 und ein freies Ende 58 auf. Entsprechend weist der rechte dritte Gurtabschnitt 54 ein erstes Ende 60 und ein freies Ende 62 auf. Die freien Enden 58, 62 der beiden dritten Gurtabschnitte 52, 54 überlappen sich und sind mit Hilfe eines weiteren Magnetschlosses 64 lösbar miteinander verbunden.

Ein vierter Gurtabschnitt 66 verbindet die beiden zweiten Enden 34, 40 der ersten Gurtabschnitte 22, 24 miteinander.

Außerdem gibt es einen fünften Gurtabschnitt 68, der die beiden ersten Enden 56, 60 der dritten Gurtabschnitte 52, 54 miteinander verbindet. Wie aus der Figur ersichtlich, bilden die zweiten Gurtabschnitte 36, 44 gemeinsam mit dem vierten Gurtabschnitt 66 und dem fünften Gurtabschnitt 68 ein Viereck, insbesondere ein Parallelogramm, insbesondere wenn die zweiten Gurtabschnitte 36, 44 gestreckt werden.

Im gezeigten Ausführungsbeispiel werden der linke erste Gurtabschnitt 22, der linke zweite Gurtabschnitt 36 und der linke dritte Gurtabschnitt 52 von einem durchgängig vom ersten Ende 26 des linken ersten Gurtabschnitts 22 bis zum freien Ende 58 des linken dritten Gurtabschnitts 52 verlaufenden, linken Hauptgurt gebildet. Entsprechend werden der rechte erste Gurtabschnitt 24, der rechte zweite Gurtabschnitt 44 und der rechte dritte Gurtabschnitt 54 von einem durchgängig vom ersten Ende 32 des rechten ersten Gurtabschnitts 24 bis zum freien Ende 62 des rechten dritten Gurtabschnitts 54 verlaufenden, rechten Hauptgurt gebildet.

Der vierte Gurtabschnitt 66 ist hingegen mit den beiden Hauptgurten vernäht, wie in der Figur durch jeweils fünf kleine parallele Striche angedeutet. Der Bereich, in dem der vierte Gurtabschnitt 66 mit den beiden Hauptgurten vernäht ist, befindet sich im Bereich des zweiten Endes 34 des linken ersten Gurtabschnitts 22 bzw. im Bereich des zweiten Endes 40 des rechten ersten Gurtabschnitts 24.

Der fünfte Gurtabschnitt 68 ist ebenfalls mit den beiden Hauptgurten vernäht, und zwar im Bereich des ersten Endes 60 des rechten dritten Gurtabschnitts 54 und im Bereich des ersten Endes 56 des linken dritten Gurtabschnitts 52.

Wie in der Figur nicht im Einzelnen gezeigt ist, besteht der vierte Gurtabschnitt 66 im wesentlichen aus einem zweiten Materialstreifen, der sich durchgängig von einem freien Ende 30 der rechten Betthalterung 18 bis zu einem freien Ende 30 der linken Betthalterung 18 erstreckt. Der zweite Materialstreifen ist weiterhin im Bereich der ersten Gurtabschnitte 22, 24 entlang seiner Längsrichtung mit den beiden Hauptgurten, das heißt mit den ersten Gurtabschnitten 22, 24, vernäht.

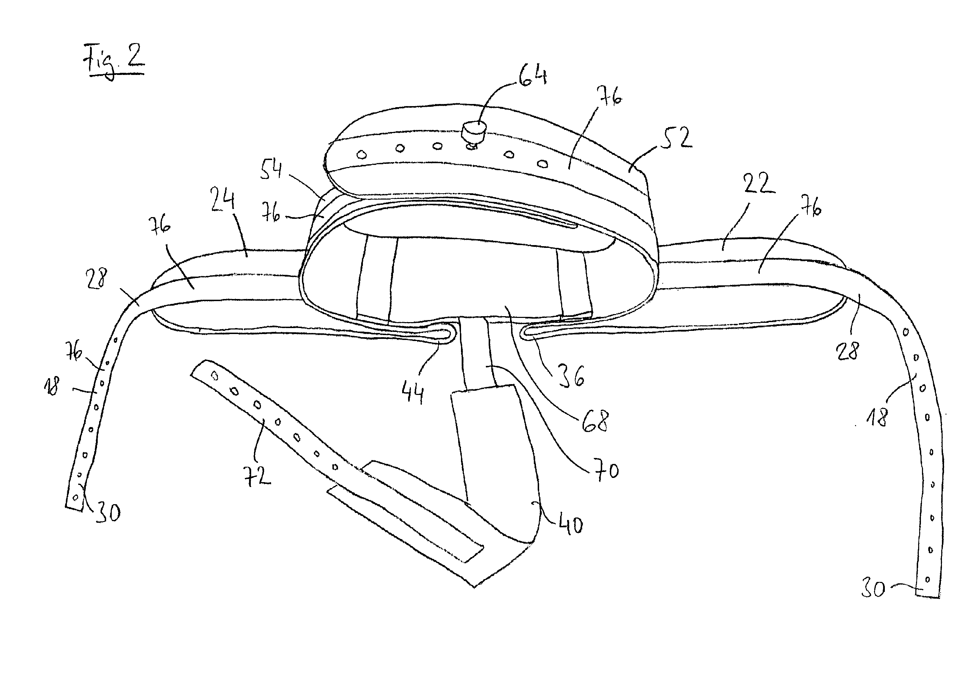

In der Figur 2 sind die bereits erläuterten Elemente der Fixierbandage mit denselben Bezugszeichen versehen. Gut erkennbar ist, dass die linken ersten, zweiten und dritten Gurtabschnitte 22, 36, 52 einen durchgängigen linken Hauptgurt bilden, und dass die rechten ersten, zweiten und dritten Gurtabschnitte 24, 44 und 54 entsprechend einen rechten, durchgängigen Hauptgurt bilden. Ebenfalls ist erkennbar, dass die genannten Gurtabschnitte bzw. von diesen gebildeten Hauptgurte eine gleichmäßige erste Breite aufweisen, die der Breite des fünften Gurtabschnitts 68 entspricht.

Außerdem zeigt Fig. 2 einen Schrittgurt 70, der an dem fünften Gurtabschnitt 68 befestigt ist und ein freies Ende 72 sowie ein Polster 74 aufweist. Das freie Ende 72 weist mehrere Ösen auf, so dass der Schrittgurt 70 mit dem Magnetschloss 64 mit den dritten Gurtabschnitten 52, 54 verbunden werden kann.

Weiterhin ist in Fig. 2 erkennbar, dass die Fixierbandage auf ihrer linken und rechten Seite jeweils einen durchgängigen ersten Materialstreifen 76 aufweist, der mit dem auf der jeweiligen Seite angeordneten Hauptgurt vernäht ist und sich durchgängig vom freien Ende 58 bzw. 68 des jeweiligen dritten Gurtabschnitts 52 bzw. 54 bis zu einem freien Ende 30 der auf der betreffenden Seite angeordneten Betthalterung 18 erstreckt.

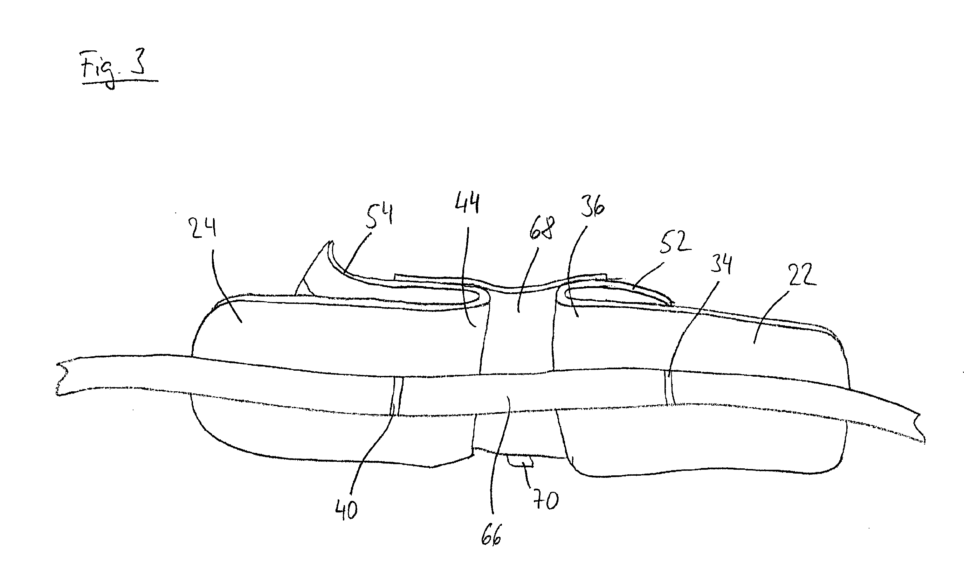

Fig. 3 zeigt einen Ausschnitt auf die Fixierbandage aus den Figuren 1 und 2 aus einem anderen Blickwinkel, nämlich ungefähr von der Liegefläche 14 aus. In dieser Ansicht ist insbesondere der vierte Gurtabschnitt 66 erkennbar und dass dieser eine zweite Breite aufweist, die geringer ist als die erste Breite beispielsweise der linken und rechten ersten Gurtabschnitte 22, 24. Die Verbindungsbereiche der vierten Gurtabschnitte 66 mit den beiden Hauptgurten definiert die Lage der zweiten Enden 34 und 40 der beiden ersten Gurtabschnitte 22 bzw. 24.

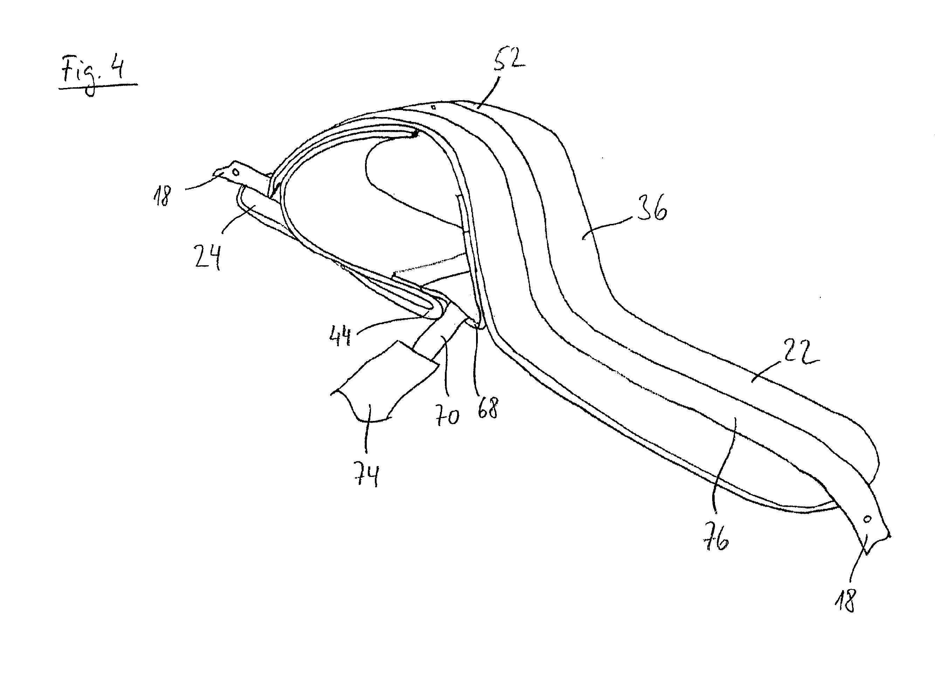

Fig. 4 zeigt einen Ausschnitt der Fixierbandage aus den Figuren 1 bis 3 in einer perspektivischen Ansicht und in einer anderen Anordnung, die etwa einem auf seine rechte Seite gedrehten Patienten entspricht. In dieser Darstellung ist gut erkennbar, dass der linke zweite Gurtabschnitt 36 gestreckt ist, so dass die auf den linken dritten Gurtabschnitt 52 ausgeübten Zugkräfte über den linken Hauptgurt bzw. den damit vernähten ersten Materialstreifen 76 bis zur linken Betthalterung 18 abgeführt werden. An dieser Darstellung ist ersichtlich, dass dem Patienten bei der erfindungsgemäßen Fixierbandage unabhängig von zusätzlichen Seitenbefestigungen ein gewisser Bewegungsspielraum verbleibt und eine seitliche Bewegung des Patienten dennoch auf äußert belastbare Weise begrenzt wird.

### Liste der verwendeten Bezugszeichen:

- 10: Matratze
- 12: Bettgestell
- 14: Liegefläche
- 16: Patient
- 18: Betthalterung
- 20: Magnetschloss
- 22: linker erster Gurtabschnitt
- 24: rechter erster Gurtabschnitt
- 26: erstes Ende des linken ersten Gurtabschnitts 22
- 28: erstes Ende der Betthalterung 18
- 30: freies Ende der Betthalterung 18
- 32: erstes Ende des rechten ersten Gurtabschnitts 24
- 34: zweites Ende des linken ersten Gurtabschnitts 22
- 36: linker zweiter Gurtabschnitt
- 38: erstes Ende des linken zweiten Gurtabschnitts 36
- 40: zweites Ende des rechten ersten Gurtabschnitts 24
- 41: zweites Ende des linken zweiten Gurtabschnitts 36
- 42: erstes Ende des rechten zweiten Gurtabschnitt 44
- 44: rechter zweiter Gurtabschnitt
- 46: zweites Ende des rechten zweiten Gurtabschnitts
- 48: erster Abstand
- 50: zweiter Abstand
- 52: linker dritter Gurtabschnitt
- 54: rechter dritter Gurtabschnitt
- 56: erstes Ende des linken dritten Gurtabschnitts 52
- 58: freies Ende des rechten dritten Gurtabschnitts 52
- 60: erstes Ende des rechten dritten Gurtabschnitts 54
- 62: freies Ende des rechten dritten Gurtabschnitts 54
- 64: Magnetschloss
- 66: vierter Gurtabschnitt
- 68: fünfter Gurtabschnitt
- 70: Schrittgurt
- 72: freies Ende des Schrittgurts70
- 74: Polster
- 76: erster Materialstreifen

## Patentansprüche

1. Fixierbandage zur Fixierung eines Patienten (16) auf einem Bett oder einem sonstigen Lager, die im angelegten Zustand folgendes aufweist:
● zwei Betthalterungen (18) zur Befestigung an einem Bettgestell (12),
● einen linken ersten Gurtabschnitt (22) und einen rechten ersten Gurtabschnitt (24), die jeweils ein erstes Ende (26, 32), das mit einer der Betthalterungen (18) verbunden ist, und ein zweites Ende (34, 40) aufweisen, wobei die beiden zweiten Enden (34, 40) in einem ersten Abstand (48) voneinander auf einer Liegefläche (14) des Betts angeordnet sind,
● einen linken zweiten Gurtabschnitt (36) und einen rechten zweiten Gurtabschnitt (44), die jeweils ein erstes Ende (38, 42) und ein zweites Ende (41, 46) aufweisen, wobei das erste Ende (38) des linken zweiten Gurtabschnitts (36) mit dem zweiten Ende (34) des linken ersten Gurtabschnitts (22) verbunden ist, das erste Ende (42) des rechten zweiten Gurtabschnitts (44) mit dem zweiten Ende (40) des rechten ersten Gurtabschnitts (24) verbunden ist und die zweiten Enden (41, 46) der beiden zweiten Gurtabschnitte (36, 44) in einem zweiten Abstand (50) voneinander im Rücken des Patienten (16) angeordnet sind,
● einen linken dritten Gurtabschnitt (52) und einen rechten dritten Gurtabschnitt (54), die jeweils ein erstes Ende (56, 60) und ein freies Ende (58, 62) aufweisen, wobei das erste Ende (56) des linken dritten Gurtabschnitts (52) mit dem zweiten Ende (41) des linken zweiten Gurtabschnitts (36) verbunden ist, das erste Ende (60) des rechten dritten Gurtabschnitts (54) mit dem zweiten Ende (46) des rechten zweiten Gurtabschnitts (44) verbunden ist und die freien Enden (58, 62) lösbar miteinander verbunden sind,
● einem vierten Gurtabschnitt (66), der die beiden zweiten Enden (34, 40) der ersten Gurtabschnitte (22, 24) miteinander verbindet, und
● einem fünften Gurtabschnitt (68), der die beiden ersten Enden (56, 60) der dritten Gurtabschnitte (52, 54) miteinander verbindet.

2. Fixierbandage nach Anspruch 1, **dadurch gekennzeichnet, dass** der fünfte Gurtabschnitt (68) und die beiden dritten Gurtabschnitte (52, 54) die gleiche Breite aufweisen.

3. Fixierbandage nach Anspruch 1 oder 2, **dadurch gekennzeichnet, dass** die ersten Gurtabschnitte (22, 24) und/oder die zweiten Gurtabschnitte (36, 44) und/oder die dritten Gurtabschnitte (52, 54) eine erste Breite aufweisen und der vierte Gurtabschnitt (66) eine zweite Breite, die geringer ist als die erste Breite.

4. Fixierbandage nach einem der Ansprüche 1 bis 3, **dadurch gekennzeichnet, dass** zwei Hauptgurte vorhanden sind, die jeweils einen der ersten Gurtabschnitte (22, 24), einen der zweiten Gurtabschnitte (36, 44) und einen der dritten Gurtabschnitte (52, 54) bilden und jeweils mindestens einen ersten Materialstreifen (76) aufweisen, der durchgängig vom ersten Ende (26, 32) des ersten Gurtabschnitts (22, 24) bis zum freien Ende (58, 62) des dritten Gurtabschnitts (52, 54) verläuft.

5. Fixierbandage nach einem der Ansprüche 1 bis 4, dass mindestens ein zweiter Materialstreifen vorhanden ist, der durchgängig von einem freien Ende (58, 62) eines der dritten Gurtabschnitte (52, 54) bis zu einem freien Ende (30) einer der Betthalterungen (18) verläuft.

6. Fixierbandage nach Anspruch 5, **dadurch gekennzeichnet, dass** der erste Materialstreifen (76) und der zweite Materialstreifen miteinander vernäht sind und der zweite Materialstreifen eine geringere Breite aufweist als der erste Materialstreifen (76).

7. Fixierbandage nach einem der Ansprüche 1 bis 6, dass an dem fünften Gurtabschnitt (68) ein Schrittgurt (70) befestigt ist.

8. Fixierbandage nach einem der Ansprüche 1 bis 7, dass an den dritten Gurtabschnitten (52, 54) oder an dem fünften Gurtabschnitt (68) eine Befestigungseinrichtung zur Anbringung eines Brust- oder Schultergurts ausgebildet ist.

9. Fixierbandage nach einem der Ansprüche 1 bis 8, dass ein Verbindungsbereich zwischen zweien der Gurtabschnitte mit einem Materialstück verstärkt ist, das mit den beiden Gurtabschnitten vernäht ist.
